# EUROPEAN PATENT SPECIFICATION

(11) **EP 2 929 037 B1**
(45) Date of publication and mention of the grant of the patent: **09.09.2020**
(21) Application number: 13823939.7
(22) Date of filing: 03.12.2013
(51) Int. Cl.: C12P 5/02, C12M 1/107, C12M 1/00

(54) **IMPROVED FERMENTATION METHOD**
VERBESSERTES FERMENTATIONSVERFAHREN
PROCÉDÉ DE FERMENTATION PERFECTIONNÉ

(30) Priority: 07.12.2012 EP 12196051
(43) Date of publication of application: 14.10.2015
(73) Proprietor: Global Bioenergies, 91000 Evry (FR)
(72) Inventor: BOCKRATH, Richard, Wilmington, Delaware 19806-1331 (US)
(74) Representative: Vossius & Partner Patentanwälte Rechtsanwälte mbB
(86) International application number: PCT/EP2013/075388
(87) International publication number: WO 2014/086780

(56) References cited:
- EP-A1- 2 468 874
- WO-A1-2011/032934
- WO-A1-2011/076691
- WO-A1-2012/052427
- BERMEJO L L ET AL: "Expression of Clostridium acetobutylicum ATCC 824 genes in Escherichia coli for acetone production and acetate detoxification", APPLIED AND ENVIRONMENTAL MICROBIOLOGY, AMERICAN SOCIETY FOR MICROBIOLOGY, US, vol. 64, no. 3, 1 March 1998 (1998-03-01), pages 1079-1085, XP002334443, ISSN: 0099-2240 cited in the application
- BIANCA N M VAN LEEUWEN ET AL: "Fermentative production of isobutene", APPLIED MICROBIOLOGY AND BIOTECHNOLOGY, SPRINGER, BERLIN, DE, vol. 93, no. 4, 11 January 2012 (2012-01-11), pages 1377-1387, XP035013024, ISSN: 1432-0614, DOI: 10.1007/S00253-011-3853-7
- STEPHANIE FOLLONIER ET AL: "Pressure to kill or pressure to boost: a review on the various effects and applications of hydrostatic pressure in bacterial biotechnology", APPLIED MICROBIOLOGY AND BIOTECHNOLOGY, SPRINGER, BERLIN, DE, vol. 93, no. 5, 31 January 2012 (2012-01-31), pages 1805-1815, XP035019256, ISSN: 1432-0614, DOI: 10.1007/S00253-011-3854-6

## Description

The present invention relates to a fermentation method for producing a hydrocarbon compound comprising the culturing of an organism in a liquid fermentation medium, wherein said organism produces a desired hydrocarbon compound by an enzymatic pathway, said enzymatic pathway comprising an intermediate which evaporates into the gaseous phase and wherein said intermediate is recovered from the gaseous phase and is reintroduced into the liquid fermentation medium, wherein said intermediate is selected from an aldehyde, a ketone and an alcohol.
Fermentation processes play an important role in the provision of various compounds and often constitute an important alternative to chemical processes. Since the operating costs of fermentation processes can be immense, there is a constant need to provide more cost efficient processes. Attempts to decrease operating costs include, e.g., the development of recombinant organisms which use a metabolic pathway for the production of the desired compound showing a reduced energy consumption, development of enzymes with higher activities, increasing efficiency in substrate conversion, the use of cheaper substrates and the like. Over the last decade attempts to produce hydrocarbon compounds which are of interest for the industry (e.g. as biofuels or as components of polymers) with the help of fermentation processes have increased and in the meantime various enzymatic reactions for the production of such compounds in microorganisms have been described. The development of corresponding fermentation methods often involves its own problems since they often make use of completely new metabolic pathways involving unusual enzymatic conversions.
The present invention addresses the need of providing improved fermentation methods for producing hydrocarbon compounds by providing the embodiments as described in the claims.

In particular, the present invention relates to a fermentation method for producing a hydrocarbon compound comprising the culturing of an organism in a liquid fermentation medium, wherein said organism produces a desired hydrocarbon compound by an enzymatic pathway, said enzymatic pathway comprising an intermediate which evaporates into the gaseous phase and wherein said intermediate is recovered from the gaseous phase and is reintroduced into the liquid fermentation medium, and wherein said intermediate is selected from an aldehyde, a ketone and an alcohol.
The process according to the present invention addresses a problem which is encountered if a fermentation process makes use of a metabolic pathway involving an intermediate which, due to its chemical and physical properties, has the tendency of escaping into the gaseous phase of the fermentation arrangement under the chosen fermentation conditions. This leads on the one hand to an inefficient use of the substrate and on the other hand it reduces the amount of the intermediate in the fermentation medium or in the organism itself resulting in a lower turnover of the intermediate by the respective enzyme which uses this intermediate as a substrate. This will result in an overall reduced efficiency of the whole process due to the leakage of the intermediate and due to the reduced efficiency of the metabolic pathway lying downstream of the intermediate.
By recovering the intermediate which evaporates into the gaseous phase from said gaseous phase and reintroducing it into the fermentation, the method according to the present invention allows to compensate for the leakage of the intermediate and to keep the concentration of the intermediate high. Maintaining a high concentration of the intermediate is advantageous since it ensures that the enzyme converting the intermediate is saturated and shows a high turnover rate. Thus, the recovery of the intermediate from the gaseous phase allows to enhance the overall productivity of the fermentation method.

The term" fermentation method" as used in the present invention relates to a method comprising the conversion of organic compounds by cells in culture.

The organism employed in a fermentation method according to the present invention is characterized in that it produces a desired hydrocarbon compound by an enzymatic pathway, i.e. it produces a desired hydrocarbon compound by converting by enzymatic reactions an organic compound into the desired hydrocarbon product.

Preferably, the hydrocarbon to be produced is a compound selected from class IA flammable liquids (i.e., a compound having a flash point below 73°F (22.8°C) and a boiling point below 100°F (37.8°C)), a compound selected from class IB flammable liquids (i.e., a compound having a flash point below 73°F (22.8°C) and a boiling point greater than or equal to 100°F (37.8°C)), a compound selected from class IC flammable liquids (i.e., a compound having a flash point greater than or equal to 73°C (22.8°C) and below 100°F (37.8°C)), or a compound selected from class II combustible liquids (i.e., a compound having a flash point greater than or equal to 100°F (37.8°C) and below 140°F (60°C)), as defined in the U.S. National Fire Protection Association's publication "NFPA 30, Flammable and Combustible Liquids Code, 2012 edition" (including the definitions of flash point and boiling point provided therein). More preferably, the hydrocarbon is a compound selected from class IA flammable liquids, class IB flammable liquids, or class IC flammable liquids.

The hydrocarbon may, for example, be an alkene, and preferably is a C₂₋₈ alkene of the following formula (I): 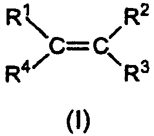 wherein R¹, R², R³ and R⁴ are each independently selected from hydrogen, C₁₋₆ alkyl or C₂₋₆ alkenyl, provided that the total number of carbon atoms in the compound of formula (I) is an integer of 2 to 8, and preferably an integer of 2 to 6 (i.e., 2, 3, 4, 5 or 6).

Preferably, the hydrocarbon is an alkene selected from ethene, propene, butene (e.g., isobutene, 1-butene or 2-butene), pentene, hexene, heptene, octene (e.g., 1-octene), butadiene (e.g., 1,3-butadiene), pentadiene (e.g., isoprene or 2-methyl-1,3-butadiene), hexadiene (e.g., 2,3-dimethyl-1,3-butadiene), heptadiene, or octadiene. More preferably, the hydrocarbon is ethene, propene, isobutene, 1-butene, 2-butene (e.g., cis-2-butene, trans-2-butene or a mixture thereof), 1,3-butadiene, or isoprene. Particularly preferred products are isobutene, 1,3-butadiene, isoprene (2-methyl-1,3-butadiene) or propene.

Even more preferably, the hydrocarbon is isobutene or 1,3-butadiene, and most preferably it is isobutene.

As used herein, the term "hydrocarbon" refers to a compound which consists of carbon atoms and hydrogen atoms and may be saturated or unsaturated, linear, branched or cyclic, aliphatic or aromatic.

The term "alkene" refers to an unsaturated aliphatic (i.e., non-aromatic) acyclic hydrocarbon which may be linear or branched and comprises at least one (e.g., one or two) carbon-to-carbon double bond while it does not comprise any carbon-to-carbon triple bond. A "C₂₋₈ alkene" refers an alkene having 2 to 8 carbon atoms. Specifically included in this definition are also alkenes having two carbon-to-carbon double bonds (i.e., alkadienes), such as, e.g., 1,3-butadiene or isoprene.

The term "alkyl" refers to a monovalent saturated aliphatic acyclic hydrocarbon group (i.e., a group consisting of carbon atoms and hydrogen atoms) which may be linear or branched and does not comprise any carbon-to-carbon double bond or any carbon-to-carbon triple bond.

The term "alkenyl" refers to a monovalent unsaturated aliphatic acyclic hydrocarbon group which may be linear or branched and comprises at least one carbon-to-carbon double bond while it does not comprise any carbon-to-carbon triple bond.

The organism employed in a fermentation method according to the present invention is furthermore characterized in that the enzymatic pathway which leads to the desired product comprises an intermediate which evaporates into the gaseous phase. In the scope of the present invention this means that under the chosen fermentation conditions the intermediate tends to pass from the liquid phase into the gaseous phase and thus significant amounts are no longer available for an enzymatic conversion in the liquid phase. Such fermentation conditions comprise preferably the cultivation of the organism in a liquid, aqueous fermentation medium. "Aqueous" preferably means that the fermentation medium contains as a solvent at least 90 vol.% water, more preferably at least 95% and even more preferably 100%. The cultivation conditions are moreover preferably chosen in such a manner that the temperature is between 25°C and 45°C, even more preferably between 30°C and 37°C, and the absolute pressure is kept between 1 bar and 15 bar, preferably between 1.2 and 5 bar.
An intermediate which evaporates during the fermentation process into the gaseous phase is also referred to in the scope of the present invention as being volatile.

In a preferred embodiment, an intermediate which evaporates into the gaseous phase means that the Intermediate, when present as a pure compound and/or any potential compound-water azeotrope of the intermediate, has an apparent boiling point of less than 120 °C at 1.013 bar or the apparent vapour pressure of the compound at infinite dilution in water at 25°C, which is defined as the pure component vapour pressure multiplied by the water-intermediate activity coefficient at infinite water dilution, is greater than 10 millibar.

Even more preferably, an intermediate which evaporates into the gaseous phase means that the intermediate when present as a pure compound and/or any potential compound-water azeotrope of the intermediate has an apparent boiling point of less than 100°C at 1.013 bar or the apparent vapor pressure of the compound at infinite dilution in water at 25°C, which is defined as the pure component vapour pressure multiplied by the water-intermediate activity coefficient at infinite water dilution, is greater than 50 millibar.

The intermediate may be any aldehyde, ketone or alcohol which occurs as an intermediate in the enzymatic pathway to the desired product and which evaporates into the gaseous phase under the chosen fermentation conditions, and which can thus be referred to as volatile under these conditions. It is to be understood that the term "intermediate" also covers mixtures of two or more intermediates as defined herein above and as described in more detail below.

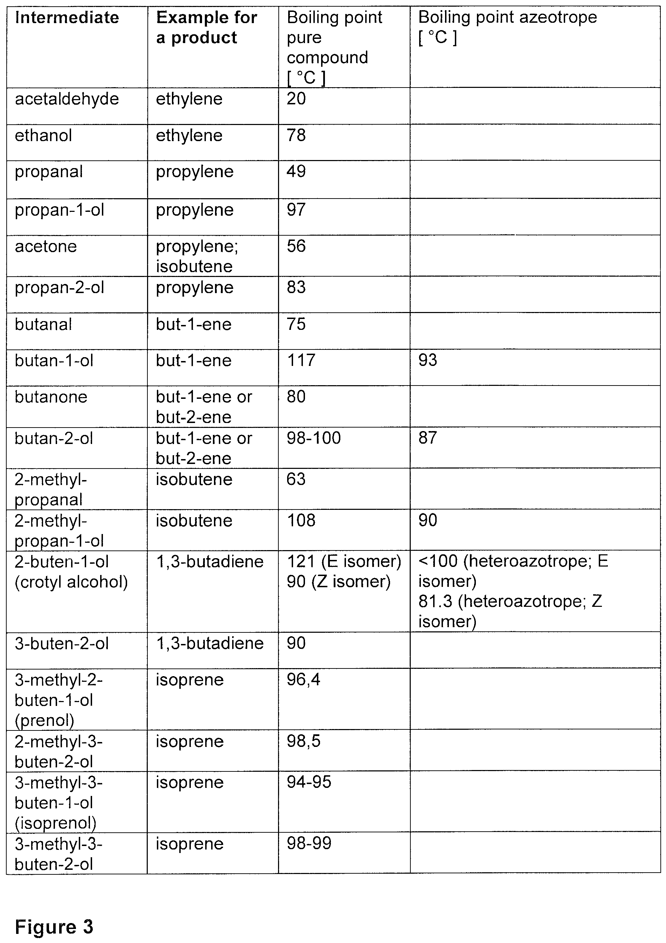

In the context of the present invention the term "intermediate" refers to compounds selected from aldehydes, ketones and alcohols. Preferred examples are selected from acetaldehyde, ethanol, acetone, propanal, propan-1-ol, propan-2-ol, butanal, butan-1-ol, butanone, butan-2-ol, 2-methyl-propanal, 2-methyl-propan-1-ol, 2-buten-1-ol (crotyl alcohol), 3-buten-2-ol, 3-methyl-2-buten-1-ol (prenol), 2-methyl-3-buten-2-ol, 3-methyl-3-buten-1-ol (isoprenol) and 3-methyl-3-buten-2-ol. The relevant physical properties of these intermediates are shown in Figure 3.
Acetaldehyde or ethanol may, for example, occur as intermediates in the production of ethylene.
Acetone, propanal, propan-1-ol or propan-2-ol may, for example, occur as intermediates in the production of propylene.
Butanal or butan-1-ol may, for example, occur as intermediates in the production of but-1-ene.
Butanone or butan-2-ol may, for example, occur as intermediates in the production of but-1-ene or but-2-ene.
Acetone, propan-2-ol, 2-methyl-propanal or 2-methyl-propan-1-ol may, for example, occur as intermediates in the production of isobutene.
2-Buten-1-ol (crotyl alcohol) or 3-buten-2-ol may, for example, occur as intermediates in the production of 1,3-butadiene.
3-Methyl-2-buten-1-ol (prenol), 2-methyl-3-buten-2-ol, 3-methyl-3-buten-1-ol (isoprenol) or 3-methyl-3-buten-2-ol may, for example, occur as intermediates in the production of isoprene.

The biological occurrence of, e.g., acetaldehyde, ethanol, acetone, butanal and butan-1-ol are well known to those skilled in the art (see Gottschalk (1986), Bacterial Metabolism, 2nd Edition, Springer-Verlag, New York) and an abundance of information on optimizing their biological production above that of naturally occurring organisms exists. More recently, the use of genetic manipulation to increase the production of propanal and propan-1-ol (Shen and Liao, Metab. Eng. 10 (2008), 312-320), propan-2-ol (Hanai et al., Appl. Environ. Microbiol. 73 (2007) 7814-7818), butanone and butan-2-ol (US 2009/0155870), 2-methyl-propanal and 2-methyl-propan-1-ol (US 7,993,889), 2-buten-1-ol (crotyl alcohol) (WO 2012/106516) and 3-methyl-2-buten-1-ol (prenol) and 3-methyl-3-buten-1-ol (isoprenol) (Withers et al., Appl. Environ. Microbiol. 73 (2007) 6277-6283; WO 2011/076261) has been reported.
Enzyme catalyzed dehydrations of ethanol to ethylene, propan-1-ol and propan-2-ol to propylene, butan-1-ol to but-1-ene, butan-2-ol to but-1-ene and but-2-ene, 2-methyl-propan-1-ol to isobutene, 2-buten-1-ol (crotyl alcohol) and 3-buten-2-ol to 1,3-butadiene, and 3-methyl-2-buten-1-ol (prenol) and 3-methyl-3-buten-1-ol (isoprenol) to isoprene (2-methyl-1,3-butadiene) have been described using a hydratase enzyme. In particular, the use of an oleate hydratase (EC 4.2.1.53) or an enzyme derived from an oleate hydratase (WO 2011/076691) or a prenyl isoflavonoid hydratase (e.g. a kievitone hydratase (EC 4.2.1.95) or a phaseollidin hydratase (EC 4.2.1.97) or an enzyme derived from a prenyl isoflavonoid hydratase (WO 2011/076689 and WO2011/076691) is described. An enzymatic conversion of 2-buten-1-ol (crotyl alcohol) to 1,3-butadiene utilizing a crotyl alcohol kinase, a 2-butenyl-4-phosphate kinase, and a butadiene synthase has been described (WO 2012/106516).

In a preferred embodiment the intermediate is non-toxic or toxic only at very high concentrations for the organism used for the fermentation method according to the present invention.

A further preferred embodiment of an intermediate according to the present invention is acetone. Acetone can be naturally produced by various organisms, including bacteria from the genus Clostridium, Bacillus or Pseudomonas, such as Clostridium acetobutylicum, Clostridium beijerinckii, Clostridium cellulolyticum, Bacillus polymyxa or Pseudomonas putida. In the meantime also recombinant organisms, for example genetically modified E. coli cells, have been reported which have the capacity to synthesize acetone (Bermejo et al., Appl. Environ. Microbiol. 64 (1998); 1079-1085; Hanai et al., Appl. Environ. Microbiol. 73 (2007), 7814-7818). Moreover, artificially created metabolic pathways have been described which make use of acetone as an intermediate in order to produce a compound of interest. For example, WO 2011/032934 describes an enzymatic method for the production of 3-hydroxy-3-methylbutyric acid (also referred to as beta-hydroxyisovalerate or HIV) from acetone and a compound which provides an activated acetyl group. The produced 3-hydroxy-3-methylbutyric acid can then be further converted into isobutene via an enzymatically catalyzed phosphorylation/decarboxylation reaction (WO 2010/001078 and WO 2012/052427).

In a preferred embodiment of the method according to the invention, the intermediate is acetone and the enzymatic pathway for the production of the desired hydrocarbon compound involves the enzymatic conversion of acetone and acetyl-CoA into 3-hydroxy-3-methylbutyric acid. This conversion can be achieved as described in WO 2011/032934. For example, the enzymatic conversion of acetone into 3-hydroxy-3-methylbutyric acid can be achieved by employing an enzyme having the activity of a HMG CoA synthase (EC 2.3.3.10) or an enzyme having the activity of a C-C bond cleavage/condensation lyase, such as a HMG CoA lyase (EC 4.1.3.4), or a PksG protein. In a preferred embodiment a HMG CoA synthase (EC 2.3.3.10) is employed, even more preferably a HMG CoA synthase from Mus musculus. A preferred example is the mitochondrial HMG CoA synthase from Mus musculus (UniProt accession number P54869 and Hegardt, Biochem. J. 338 (1999), 569-582).

In a preferred embodiment according to the present invention, the intermediate is acetone and the hydrocarbon compound to be produced by said enzymatic pathway is isobutene. Preferably the synthesis of isobutene is achieved by first enzymatically converting acetone and acetyl-CoA into 3-hydroxy-3-methylbutyric acid and then further converting 3-hydroxy-3-methylbutyric acid into isobutene in two steps. The former, reaction of 3-hydroxy-3-methylbutyric acid with ATP to form 3-methyl-3-phosphonoxy-butyric acid (3-phosphonoxy-isovaleric acid) is preferably achieved by an enzymatically catalysed phosphorylation reaction as described in WO 2012/052427. The latter conversion is preferably achieved by an enzymatically catalyzed decarboxylation reaction as described, e.g., in WO 2010/001078 and WO 2012/052427. It is most preferred that the decarboxylation of 3-hydroxy-3-methylbutyric acid into isobutene is achieved by the use of a mevalonate diphosphate decarboxylases.

In another preferred embodiment according to the present invention, the intermediate is acetone and the hydrocarbon compound to be produced by said enzymatic pathway is propylene.

Preferably the synthesis of propylene is achieved by first enzymatically converting acetone into propan-2-ol by a secondary alcohol dehydrogenase (Hanai et al., Appl. Environ. Microbiol. 73 (2007) 7814-7818). In a subsequent step, the propan-2-ol is dehydrated to propylene by a hydratase enzyme as described, e.g. in WO 2011/076691 and WO 2011/076689. It is preferred that the dehydration of propan-2-ol into propylene is achieved by a oleate hydratase (EC 4.2.1.53), kievitone hydratase (EC 4.2.1.95), or phaseollidin hydratase (EC 4.2.1.97).

In another preferred embodiment according to the present invention, the intermediate is 2-buten-1-ol (crotyl alcohol) or 3-buten-2-ol and the hydrocarbon compound to be produced by said enzymatic pathway is 1,3-butadiene. Preferably the synthesis of 1,3-butadiene is achieved by enzymatically converting 2-buten-1-ol (crotyl alcohol) or 3-buten-2-ol into 1,3-butadiene. The conversion 3-buten-2-ol into 1,3-butadiene is preferably achieved by an enzymatically catalyzed dehydration reaction. It is most preferred that the conversion is achieved by the use of an oleate hydratase (EC 4.2.1.53), kievitone hydratase (EC 4.2.1.95), or phaseollidin hydratase (EC 4.2.1.97). The conversion of 2-buten-1-ol (crotyl alcohol) into 1,3-butadiene is preferably achieved by the use of a crotyl alcohol kinase, a 2-butenyl-4-phosphate kinase, and a butadiene synthase (WO 2012/106516).

In another preferred embodiment according to the present invention, the intermediate is 3-methyl-2-buten-1-ol (prenol), 2-methyl-3-buten-2-ol, 3-methyl-3-buten-1-ol (isoprenol) or 3-methyl-3-buten-2-ol and the hydrocarbon compound to be produced by said enzymatic pathway is isoprene. Preferably the synthesis of isoprene is achieved by enzymatically converting 3-methyl-2-buten-1-ol (prenol), 2-methyl-3-buten-2-ol, 3-methyl-3-buten-1-ol (isoprenol) or 3-methyl-3-buten-2-ol into isoprene. The conversions are preferably achieved by an enzymatically catalyzed dehydration reaction. It is most preferred that the conversion is achieved by the use of an oleate hydratase (EC 4.2.1.53), kievitone hydratase (EC 4.2.1.95), or phaseollidin hydratase (EC 4.2.1.97).

The organism employed in the method according to the invention is preferably a microorganism, more preferably a unicellular microorganism. In another preferred embodiment the organism is a cell.
The cells employed in the fermentation method according to the present invention may be any cells which can be cultivated in a fermentation medium, e.g. animal cells, plant cells, bacterial cells or fungal cells. Preferably the cells are bacterial cells or fungal cells. Preferred bacterial cells are cells of bacteria of the genus Escherichia, Zymomonas, Rhodococcus, Pseudomonas, Bacillus, Lactobacillus, Enterococcus, Pediococcus, Alcaligenes, Klebsiella, Paenibacillus, Arthrobacter, Corynebacterium or Brevibacterium. In a particularly preferred embodiment the bacterial cells are E. coli, Alcaligenes eutrophus, Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Pediococcus pentosaceus, Pediococcus acidilactici, Zymomonas mobilis, Corynebacterium glutamicum or Bacillus subtilis cells. In a most preferred embodiment the bacterial cells are E. coli cells. Preferred fungal cells are cells of the genus Saccharomyces, Pichia, Candida, Hansenula, Schizosaccharomyces or Kluyveromyces. In a particularly preferred embodiment the fungal cells are Saccharomyces cerevisiae, Pichia pastoris, Candida albicans, Hansenula polymorpha, Schizosaccharomyces pombe, Kluyveromyces marxianus or Kluyveromyces lactis cells. In a most preferred embodiment the fungal cells are S. cerevisiae cells.

Preferably, the cells are cells which are able to synthesize acetone. Preferably the cells are E. coli cells which are able to synthesize acetone, more preferably E.coli cells which are able to synthesize acetone and to convert it into 3-hydroxy-3-methylbutyric acid. Even more preferably such E. coli cells are also capable of further converting 3-hydroxy-3-methylbutyric acid into isobutene, preferably by a phosphorylation/decarboxylation reaction.

In a preferred embodiment of the method according to the present invention, the fermentation is carried out under increased pressure. The term "increased pressure" means a pressure which is higher than atmospheric pressure and is measured at the fermenter head space. Preferably, the absolute pressure used in the fermentation method is at least 1.2 bar, in particular at least 1.5 bar, more preferably at least 2 bar, even more preferably at least 4 bar, and particularly preferred at least 5 bar. There is principally no upper limit for the pressure since it is known that microbial cells such as bacterial and fungal cells can be cultured under extremely high pressure. A limiting feature is generally the design of the fermentation assembly since high pressure makes necessary the reinforcement of the different parts of the fermentation assembly. Correspondingly, the pressure generally does not exceed 50 bar, preferably 30 bar, more preferably 25 bar, even more preferably 20 bar, particularly preferred 15 bar. In a preferred embodiment the pressure is between 1.2 bar and 25 bar, even more preferably between 1.5 and 20 bar.

The use of increased pressure during fermentation has the advantage that less intermediate is lost into the gaseous phase and has to be recovered from the gaseous phase so as to be reintroduced into the fermentation process. This can lead to a considerable reduction of costs. For example, if the pressure is doubled, the amount of volatile intermediate which escapes into the gaseous phase is reduced by 50% and, consequently, only half of the amount of volatile intermediate has to be recovered from the gaseous phase which can dramatically reduce the overall costs. Furthermore, as will be set out below, an increased pressure in the fermenter can facilitate the downstream processing of the fermentation off-gas, in particular the recovery of the intermediate and/or the hydrocarbon from a gaseous stream leaving the fermenter.

In a preferred embodiment the method according to the present invention is carried out under aerobic conditions. When using aerobic conditions the gas flow-rates are usually higher than under anaerobic conditions. The flow rate of the inlet gas corresponds to the volume of inlet gas which is fed into the fermenter per unit time. If the flow rate of the inlet gas is increased, the oxygen concentration in the fermentation off-gas will also increase because more oxygen comprised in the inlet gas will pass through the fermenter without being consumed by the microorganism, and vice versa. Typical inlet gas flow rates may, for example, lie in the range of about 0.05 vvm to about 0.5 vvm (vessel volumes per minute, i.e. volume of inlet gas flow under standard conditions (101.325 kPa, 20°C) per volume of liquid fermentation medium per minute) and will depend, *inter alia,* on the sugar concentration in the fermentation medium, the concentration of the microorganism in the medium, the reaction pathway requirements, and the specific rate of sugar consumption per unit concentration of microorganism per unit time.
The inlet-gas can, e.g., be air or air which is supplemented with oxygen so as to obtain a higher partial pressure of oxygen. The partial pressure of oxygen in the inlet gas can be adjusted in different ways, e.g., by adjusting the oxygen concentration (vol-% O₂) in the inlet gas and/or by adjusting the total pressure of the inlet gas before introduction into the fermenter. In the latter case, the adjustment of the partial pressure of oxygen in the inlet gas is effected by increasing or decreasing the total pressure of the inlet gas before introduction into the fermenter. A higher total pressure of the inlet gas before introduction into the fermenter will lead to a correspondingly increased system pressure in the fermenter and will thereby increase the transfer of oxygen from the inlet gas into the liquid fermentation medium, resulting in a decreased oxygen concentration in the fermentation off-gas. Also, increasing the concentration of oxygen dissolved in the liquid fermentation medium may stimulate the growth of the microorganism and the fermentative production of the hydrocarbon by the microorganism. The partial pressure of oxygen in the inlet gas can also be adjusted by increasing or decreasing the oxygen concentration in the inlet gas. This can be achieved, e.g., by mixing or co-feeding the inlet gas with another gas having either a lower or a higher oxygen concentration than the inlet gas (for example, by admixing an inert gas, such as nitrogen, argon or fermentation gas in which the desired hydrocarbon product has been largely or completely removed, to decrease the oxygen concentration in the inlet gas, or by admixing oxygen-enriched air or pure oxygen to increase the oxygen concentration in the inlet gas). The adjustment of the oxygen concentration in the inlet gas by admixing an inert gas is less advantageous in terms of cost effectiveness. The oxygen concentration in the inlet gas can also be adjusted by either removing a fraction of an inert gas (e.g., nitrogen) from the inlet gas using a corresponding membrane (e.g., a nitrogen removal membrane) or removing a fraction of oxygen from the inlet gas using an oxygen removal membrane. Suitable gas separation membranes are described, e.g., in Koros WJ et al., Journal of Membrane Science, 1993, 83(1):1-80; Koros WJ et al., Journal of Membrane Science, 2000, 175(2):181-196; Robeson LM, Current Opinion in Solid State and Materials Science, 1999, 4(6):549-552; Yampolskii Y, Macromolecules, 2012, 45(8):3298-3311; Baker RW, Ind Eng Chem Res, 2002, 41(6):1393-1411; Bernardo P et al., Ind Eng Chem Res, 2009, 48(10):4638-4663; and Ismail AF et al., Journal of Membrane Science, 2001, 193(1):1-18.

According to the present invention, the intermediate which evaporates into the gaseous phase is recovered and reintroduced into the liquid fermentation medium in order to maintain a high concentration of said intermediate in the medium/cells and to thereby ensure that the intermediate is efficiently further converted enzymatically.
Methods for recovering gaseous substances, such as acetone, from gaseous mixtures are known to those skilled in the art and comprise but are not limited to:
- Physical absorption with or without distillation;
- Reactive absorption with or without distillation;
- Adsorption
- Condensation;
- Cryogenic technologies; and
- Membrane based separations.

Also the hydrocarbon can also be recovered or isolated from the fermentation off-gas using techniques known in the art, such as, e.g., physical absorption with or without distillation, reactive absorption with or without distillation, adsorption, condensation, cryogenic technologies, and/or membrane-based separation. For example, hydrocarbon and intermediate evaporating into the gaseous phase during the fermentation process can be withdrawn from the fermenter as a mixture in the fermentation off-gas, and are subsequently recovered and separated so as to allow the reintroduction of the intermediate into the liquid fermentation medium and the purification of the hydrocarbons.

In accordance with a preferred embodiment of the invention, the recovery of the intermediate from the gaseous phase is accomplished via a recovery process which comprises:
a. contacting a fermentation off-gas containing the hydrocarbon as the desired product and the intermediate with a liquid absorbent to absorb the desired product and the intermediate in the liquid absorbent;
b. desorbing the desired product and the intermediate by desorption from the liquid absorbent; and
c. separating the desired product and the intermediate product to recover the desired product and the intermediate.

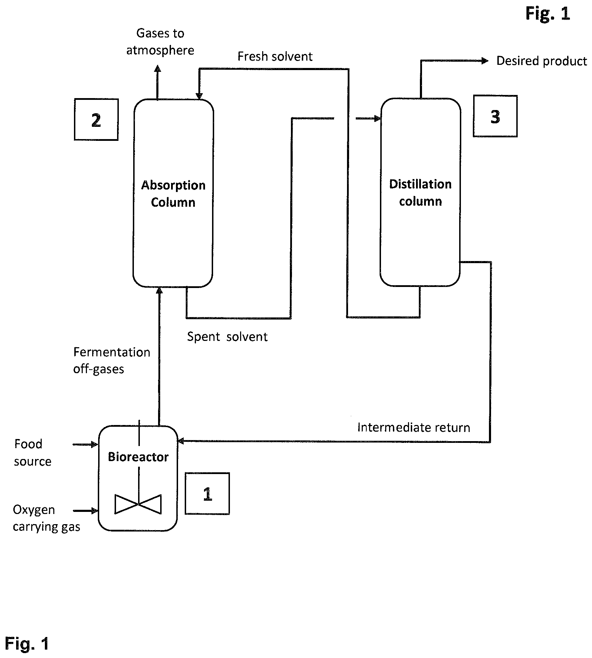

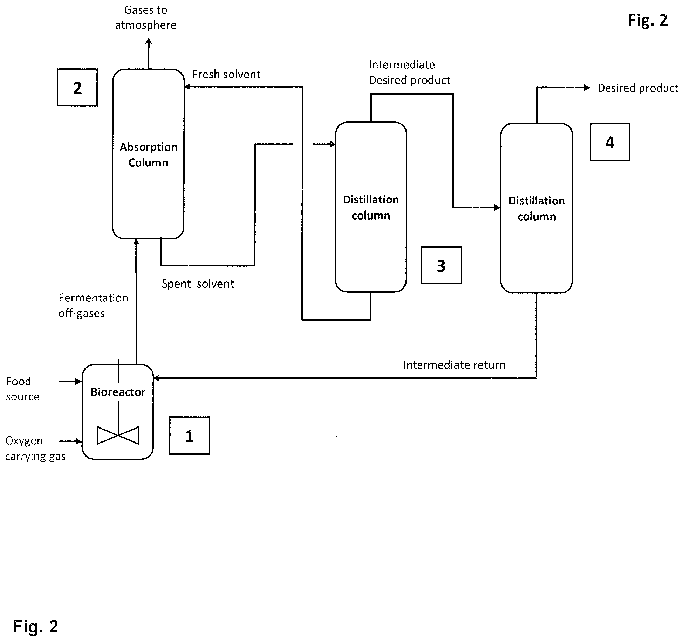

As illustrated in the exemplary embodiments of Fig. 1 and Fig. 2, the contact between the fermentation off-gas and the liquid absorbent can be achieved in an absorption device (2) separate from the fermentation device (1). For example, an absorption column can be used.

Exemplary liquid absorbents are selected from: (i) linear or branched C₁₀-C₂₀ alkanes; (ii) linear or branched C₁₀-C₂₀ monoalkenes; (iii) polyglycol ethers, e.g. those of the formula R⁵-[O-R⁶]ₘ-OR⁷, wherein R⁵ is selected from hydrogen and C₁₋₆ alkyl, said alkyl being optionally substituted by a hydroxy or a carboxy group, R⁶ is selected from ethylene, linear or branched propylene, and linear or branched butylene, m represents an integer from 2 to 5, and R⁷ is selected from hydrogen and C₁₋₆ alkyl, said alkyl being optionally substituted by a hydroxy or a carboxy group; (iv) mixtures of two or more of the above; (v) phenyl ethers, including diphenyl ethers and eutectic mixtures of phenyl ethers which are liquid at 25 °C with phenyl ethers of the formula Ph-OR⁸, wherein Ph represents a phenyl group and R⁸ represents phenyl, optionally substituted with one or more (such as one, two or three) C₁₋₅ alkyl groups; (vi) alcohols of the formula R⁹-OH, wherein R⁹ represent a linear or branched C₆ to C₁₅ alkyl group, preferably a C₇ to C₁₂ alkyl group; and (vii) aromatic solvents and alkylated aromatics, including solvents of the formula Ph-[CH₂]ₙ-Ph, wherein Ph is Phenyl and n is an integer from 1 to 5, and alkylated aromatic compounds, in particular those with a phenyl or naphtyl aromatic system which carries one or more, such as one, two or three, C₁₋₅ alkyl substitutents. If desired, additives such as an anti-oxidant may be added to the liquid absorbent.

The fermentation off-gas (i.e. a gas stream originating from the fermenter) typically comprises the hydrocarbon as the desired product and the intermediate together with additional gaseous components. Generally, the total content of the desired product, such as isobutene, and the intermediate, such as acetone, in the fermentation off-gas is in a range of 3 to 30 vol.%, preferably 3 to 20 vol.%. The remaining gaseous components are, for example, N₂, O₂ and CO₂. It is preferable that more than 90 % of the total amount of the desired product and the intermediate, more preferably more than 95 % of the desired product and the intermediate, are absorbed by the liquid absorbent. If necessary and/or desired, more than one (e.g. two) absorption devices can be combined to achieve a high degree of absorption. The absorption generally proceeds at a temperature of the liquid absorbent of -10 to 50 °C, preferably 5 to 35 °C. Moreover, it is generally useful in terms of the absorption efficiency if the fermentation off-gas subjected to the absorption step has an increased pressure, e.g. an absolute pressure of about 1.2 to about 50 bar. The optimum pressure may vary depending on the desired product. For C3 hydrocarbons, a pressure range of about 2 to about 20 bar is preferred, for C4 hydrocarbons, such as isobutene, a pressure range of 2 to 10 bar is preferred.

Also in this respect, carrying out the fermentation under increased pressure as indicated above as a preferred approach in the context of the invention is advantageous as it avoids the need for compressing a potentially larger volume of fermentation off-gas which contains a hydrocarbon. Preferably, the increased pressure under which the fermentation is carried out is the same, or substantially the same, as the pressure under which the absorption of the desired product and the intermediate is carried out. If, for example, the hydrocarbon to be produced is isobutene, the subsequent recovery/isolation of isobutene from the fermentation off-gas is preferably carried out under a pressure of about 2 to about 10 bar, e.g. 4 bar. Thus, if the fermentation is carried out at about the same pressure the resulting fermentation off-gas can be further processed in order to isolate the desired isobutene without having to compress the fermentation off-gas. This is particularly advantageous since the fermentation off-gas containing the flammable hydrocarbon does not need to be further compressed for subsequent isolation.

After the absorber liquid has been contacted with the fermentation off-gas and has absorbed the desired product and the intermediate, the liquid absorbent is generally separated from the non-absorbed residual gas. Typically, the composition of the residual gas is such that it can be vented into the atmosphere. The liquid absorbent containing the desired product and the intermediate, generally in a physically absorbed form, is allowed to proceed to the next step involving the desorption of the desired product and the intermediate.

In one exemplary embodiment of the recovery process illustrated in Fig. 1, the desorption and the separation of the intermediate and the desired product are carried out in a single distillation apparatus (3). The desired product and the intermediate are drawn off separately, e.g. the desired product can be drawn off at the top of the column, and the intermediate can be drawn off laterally. Once recovered, the intermediate can be purified further if needed, and recycled to the fermenter (1), typically as a liquid. The liquid absorbent can be recovered at the bottom of the distillation apparatus and recycled to the absorption apparatus (2). For this embodiment, a conventional side-draw distillation column can be employed. A divided wall column is an alternative to the normal distillation column to increase separation efficiency. The desired product can be further purified using distillation, condensation or gas permeation methods known in the art.

In accordance with a second exemplary embodiment of the recovery process illustrated in Fig. 2, desorption and separation of the intermediate and the desired product are carried out in two separate apparatuses as follows:
First, the liquid absorbent/intermediate/desired product mixture is introduced in a desorber (3), where a, typically gaseous, stream containing the intermediate and the desired product is separated from the liquid absorbent. For example, the intermediate and desired product can be drawn off at the top of the column. The liquid absorbent can be recovered at the bottom of the column and recycled to the absorption apparatus (2).

The intermediate/desired product stream from the desorber (3) is introduced in a distillation column (4) which separates the intermediate and the desired product. For example, the intermediate can be recovered at the bottom of the distillation apparatus, purified further if needed, and recycled to the fermenter (1), typically as a liquid. The desired product is drawn off e.g. at the top of the distillation apparatus (4). It can be further purified using distillation, condensation or gas permeation methods known in the art.

In both embodiments, desorption of intermediate and desired product can be achieved by normal distillation or by using a boil-up liquid (e.g. water).

Desorption and separation steps can be carried out in a pressure range of 1 to 50 bar, more preferably of 1 to 20 bar, even more preferably of 1 to 10 bar.

If desired, it is possible to recover additional amounts of intermediate and/or desired product which do not enter the gas phase and leave the fermenter in the fermentation off-gas, but remain dissolved in the liquid fermentation broth, especially at the end of a fermentation cycle. For example, this can be accomplished by a conventional distillation procedure to separate the desired product and/or the intermediate from the fermentation broth (liquid). Desired product and/or intermediate can then be (re)used.

The fermentation method according to the present invention is carried out by culturing the organism in a liquid fermentation medium. A fermentation medium is used which allows growth of the organism to be cultured and the production of the hydrocarbon compound to be produced. The choice of the appropriate culture medium belongs to the common general knowledge of the person skilled in the art.
Generally, a fermentation medium contains a suitable carbon source. Examples of suitable carbon sources are, but are not restricted to, monosaccharides such as glucose and fructose, oligosaccharides such as lactose or sucrose, polysaccharides such as starch or cellulose or mixtures thereof and unpurified mixtures from renewable feedstocks such as cheese whey permeate, cornsteep liquor, sugar beet molasses, and barley malt. The carbon source may also be a one-carbon substrate, such as carbon dioxide or methanol for which metabolic conversion into key biochemical intermediates has been demonstrated. For example, glycerol production from single carbon sources (e.g., methanol, formaldehyde or formate) has been reported in methylotrophic yeasts (K. Yamada et al., Agric. Biol. Chem. 53 (1989), 541-543) and in bacteria (Hunter et. al., Biochemistry 24 (1985), 4148-4155). These organisms can assimilate single carbon compounds, ranging in oxidation state from methane to formate, and produce glycerol. The pathway of carbon assimilation can be through ribulose monophosphate, through serine, or through xylulose-momophosphate (Gottschalk, Bacterial Metabolism, Second Edition, Springer-Verlag: New York (1986)). The ribulose monophosphate pathway involves the condensation of formate with ribulose-5-phosphate to form a 6 carbon sugar that becomes fructose and eventually the three carbon product glyceraldehyde-3-phosphate. Likewise, the serine pathway assimilates the one-carbon compound into the glycolytic pathway via methylenetetrahydrofolate.
Some organisms, such as methylotrophic organisms, are also known to utilize a number of other carbon containing compounds such as methylamine, glucosamine and a variety of amino acids for metabolic activity. For example, methylotrophic yeasts are known to utilize the carbon from methylamine to form trehalose or glycerol (Bellion et al., Microb. Growth C1 Compd., [Int. Symp.], 7th (1993), 415-432. Editor(s): Murrell, J. Collin; Kelly, Don P. Publisher: Intercept, Andover, UK). Similarly, various species of Candida metabolize alanine or oleic acid (Sulter et al., Arch. Microbiol. 153 (1990), 485-489). Therefore, the carbon source contained in the fermentation in the process according to the present invention may encompass a wide variety of carbon containing substrates and will only be limited by the choice of organism and possibly by the choice of the desired product to be produced.

Preferred carbon sources are glucose, fructose, sucrose, sugar mixtures derived from cellulosic or lignocellulosic feedstocks, or methanol. Most preferably glucose is used s a carbon source.

In addition to an appropriate carbon source, the fermentation medium generally also contains suitable minerals, salts, cofactors, buffers and other components, known to those skilled in the art, suitable for the growth of the cultures and promotion of the enzymatic pathway necessary for the production of the desired compound to be produced.

The fermentation medium used is not critical, but it must support growth of the organism used and promote the biosynthetic pathway necessary to produce the desired product. A conventional fermentation medium may be used, including, but not limited to, complex media containing organic nitrogen sources such as yeast extract or peptone and at least one fermentable carbon source; minimal media; and defined media. Preferred growth media in the present invention are common commercially prepared media such as Luria Bertani (LB) broth, Sabouraud Dextrose (SD) broth or Yeast medium (YM) broth. Other defined or synthetic growth media may also be used and the appropriate medium for growth of the particular microorganism will be known by someone skilled in the art of microbiology or fermentation science. The pH value of the fermentation medium is adjusted to meet the growth requirements of the organism employed in the method. Suitable pH ranges for the fermentation are between pH 5.0 to pH 9.0 where pH 6.0 to pH 8.0 is preferred as the initial condition.
The fermentation can be carried under aerobic or anaerobic conditions.

The cells contained in the fermentation medium are grown at a suitable temperature depending on the requirements of the cells. Typical fermentation conditions employ temperatures of around 30 °C to 37 °C. However, if thermophilic organisms are employed in the fermentation method according to the present invention, higher temperatures may be used.

The fermentation method according to the present invention may be carried out in any suitable fermenter. Such fermenters include a stirred tank fermenter, an airlift fermenter, a bubble column fermenter or any combination thereof. The fermentation arrangement is done in a way that it is possible to recover the gaseous phase present in the fermenter into which the volatile intermediate evaporates.

Typically and preferably the fermentation method is carried out as a batch method. A classical batch fermentation is a closed system where the composition of the media is set at the beginning of the fermentation and is not subject to artificial alterations during the fermentation. Thus, at the beginning of the fermentation the medium is inoculated with the desired organism or organisms and fermentation is permitted to occur adding nothing to the system apart from the volatile intermediate which is recovered from the gaseous phase and is reintroduced into the liquid fermentation medium. Typically, however, a "batch" fermentation is batch with respect to the addition of carbon source and attempts are often made at controlling factors such as pH and oxygen concentration. In batch systems the metabolite and biomass compositions of the system change constantly up to the time the fermentation is stopped. Within batch cultures cells moderate through a static lag phase to a high growth log phase and finally to a stationary phase where growth rate is diminished or halted. If untreated, cells in the stationary phase will eventually die. Cells in log phase generally are responsible for the bulk of production of end product or intermediate. The fermentation method according to the present invention may also be designed as a Fed-Batch method. A Fed-Batch method comprises a typical batch system with the exception that the substrate is added in increments as the fermentation progresses. Fed-Batch systems are useful when catabolite repression is apt to inhibit the metabolism of the cells and where it is desirable to have limited amounts of substrate in the media. Batch and Fed-Batch fermentations are common and well known in the art and examples may be found in Thomas D. Brock in Biotechnology: A Textbook of Industrial Microbiology, Second Edition (1989) Sinauer Associates, Inc., Sunderland, Mass., or Deshpande, Mukund V., Appl. Biochem. Biotechnol., 36, 227, (1992). Although the present invention is preferably carried out in batch mode it is in principle also possible that the method is carried out by using continuous fermentation. Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth. One advantage of continuous fermentation is that it allows for the modulation of one factor or any number of factors that affect cell growth or end product concentration. By continuous fermentation it is attempted to maintain steady state growth conditions and thus the cell loss due to media being drawn off must be balanced against the cell growth rate in the fermentation. Methods of modulating nutrients and growth factors for continuous fermentation processes as well as techniques for maximizing the rate of product formation are well known in the art of industrial microbiology and a variety of methods are detailed by Brock (loc. cit).
- **Figure 1**: shows schematically a possible fermentation arrangement to be used in the context of the present invention
- **Figure 2**: shows schematically an alternative possible fermentation arrangement to be used in the context of the present invention
- **Figure 3**: shows an overview of preferred intermediates and their physical properties.
- **Figure 4**: shows the positive impact of the volatile intermediate acetone on the formation of the product isobutene (IBN) and the key pathway compound 3-hydroxyisovalerate (HIV).
- **Figure 5**: shows the results of the experiments described in Example 10 concerning the effect of pressure during fermentation on the concentration of the volatile intermediate acetone.

It is to be understood that the present invention specifically relates to each and every combination of features and process parameters described herein, including any combination of general and/or preferred features/parameters. In particular, the invention specifically relates to all combinations of preferred features (including all degrees of preference) of the process provided herein.

In this specification, a number of documents including patent applications are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention will now be described by reference to the following examples which are merely illustrative and are not to be construed as a limitation of the scope of the present invention.

### Examples

### Example 1: Biological Production of Acetone

A recombinant E. coli strain, derived from MG1655 (ATCC Number 700926; American Type Culture Collection (ATCC), Manassas, VA), was used for the production of acetone. The strain, designated GBE2273, comprised a host cell (GBE1518) and a plasmid (pGBE1020). Strain GBE1518 comprised a modified central carbon metabolism network which employed a phosphoketolase pathway to more effectively channel carbon from a glucose feedstock to acetyl-coenzyme A (Ac-CoA), a key metabolite for the production of acetone. The plasmid pGBE1020 comprised genes encoding thiolase, coenzyme A transferase, and acetoacetate decarboxylase enzymes, which are responsible for catalyzing the conversion of two molecules of Ac-CoA into acetoacetyl-CoA, acetoacetyl-CoA into acetoacetate, and acetoacetate into acetone, respectively. Strain GBE2273 was inoculated at an initial OD₆₀₀ of 0.1 absorbance units (AU) in a 250 mL screw cap flask containing 200 mL of medium. The medium was MS minimal medium (Richaud et al., J. Biol. Chem. (1998), 26827-26835), supplemented with 150 mM K₂HPO₄, 0.1 g/L yeast extract, 0.1 mg/L ampicillin, and 10 g/L glucose. The flask was incubated at 30 °C and 170 rpm for 6 days. Aliquots were withdrawn every 24 hr for 6 days. The flask was kept closed to avoid escape of acetone by evaporation, except for a brief period during each sampling. Glucose was determined by HPLC analysis and acetone was determined by GC. In particular, the acetone concentration was determined by gas chromatography using Gas chromatograph 450-GC (Bruker) and the following program:
- Column : DB-WAX (123-7033, Agilent Technologies)
- Injector Split/Splitless : T° = 250°C
- Oven:
   ∘ 80°C for 6 minutes
   ∘ 10°C per minutes until 220°C
   ∘ 220°C for 7 minutes
   ∘ Column flow : 1,5ml/minute (Nitrogen)
- Detector FID : T° = 300°C

The yield of acetone was 0.51 mol acetone per mole glucose consumed.

### Example 2: Fermentative Production of Isobutene

An E. coli strain, strain MG1655 transformed with a plasmid comprising genes encoding an HMG-CoA synthase for the conversion of acetone and acetyl-CoA into 3-hydroxy-3-methylbutyric acid, a mevalonate diphosphate decarboxylase for the conversion of 3-hydroxy-3-methylbutyric acid and ATP to 3-methyl-3-phosphonoxy-butyric acid (3-phosphonoxy-isovaleric acid), and a second mevalonate diphosphate decarboxylase for the conversion of 3-methyl-3-phosphonoxy-butyric acid to isobutene was constructed. Each of these genes had previously been mutated from their parental genes in order to more effectively perform the conversions described above, see WO 2010/001078 and WO 2012/052427.

The E. coli strain was inoculated into a fermenter equipped with temperature, pH and dO (dissolved oxygen) control and substrate feed pumps at an initial OD₆₀₀ of 0.5 AU. The initial volume was 1.0 L of MS minimal medium (Richaud et al., J. Biol. Chem. (1998), 26827-26835) supplemented with 0.1 g/L yeast extract and 0.1 mg/mL ampicillin. The temperature was set for 30 °C; the pH was set to control at pH = 6.8, with ammonium hydroxide addition; the gas (air) flow rate and initial stirrer rate was set at 0.25 WM (volume per volume per minute) and 400 rpm (1200 rpm maximum), respectively; and the dO control was set for 50% with no back pressure. The fermentation was performed at atmospheric pressure. Glucose was maintained below 30 mM for 12 h by pump feed, at which time a bolus was added (approximately 45 g/L) and the fermentation was subsequently run in batch mode with respect to glucose. Acetone, initially present at approximately 100 mM, was subsequently provided by pump feed in order to make up for acetone lost by evaporation into the exit gases.

At various times, glucose, 3-hydroxy-3-methylbutyric acid and acetone in the liquid phase were determined by HPLC while acetone and isobutene in the gas phase were monitored by GC. The maximum specific isobutene productivity was 0.007 mmol/g dcw/hr with a concomitant specific 3-hydroxy-3-methylbutyric acid productivity of 0.08 mmol/g dcw/hr and a specific glucose consumption rate of 1.0 mmol/g dcw/hr, where dcw is dry cell weight.

### Example 3: Fermentative Production of Acetone and Isobutene

A recombinant E. coli strain, derived from strain MG1655, is constructed. The recombinant strain comprises a modified central carbon metabolism network employing a phosphoketolase pathway to more effectively channel carbon from a glucose feedstock to acetyl-coenzyme A (Ac-CoA); thiolase, coenzyme A transferase, and acetoacetate decarboxylase enzymes to convert Ac-CoA into acetone; and a mutated HMG-CoA synthase enzyme and mutated mevalonate diphosphate decarboxylase enzymes to convert acetone and Ac-CoA into isobutene.

The E. coli strain is inoculated into a fermenter equipped with temperature, pH and dO (dissolved oxygen) control and substrate feed pumps. The fermentation was performed at atmospheric pressure. Temperature is controlled at 32 °C, pH is controlled at 6.8, and dO is controlled at 20% by air flow (initially 0.25 VVM) and stirrer speed (initially 400 rpm) with no back pressure. The medium initially contains: KH₂PO₄, 7.5 g/L; MgSO₄.7H₂O, 2.0 g/L; citric acid, 1.83 g/L; ferric ammonium citrate, 0.3 g/L; CaCl₂.2H₂O, 0.2 g/L; sulfuric acid (98%), 1.2 mL/L; yeast extract, 5 g/L; and glucose (10 g/L). After sterilization, 10 mL of a trace metal mixture and 1 mL of a thiamine solution (150 mM) is added and the medium is adjusted to pH 6.8 with 3 M NaOH. The trace metal mixture contains: MnSO₄.H₂O, 3 g/L; NaCl, 1 g/L; FeSO₄.7H₂O, 0.1 g/L; CoCl₂.6H₂O, 0.1 g/L; ZnSO₄.7H₂O, 0.1 g/L; CuSO₄.5H₂O, 0.0064 g/L; H₃BO₃, 0.01 g/L; and Na₂MoO₄.2H₂O, 0.01 g/L.

The initial OD₆₀₀ of the fermenter is approximately 0.1 AU. Glucose is added as necessary to maintain the concentration above zero but below 10 g/L. When the OD₆₀₀ of the fermenter reaches approximate 5 AU, acetone is added to the fermenter to approximately 250 mM and this concentration is maintained throughout the course of the fermentation. Isobutene and acetone are produced. The amount of exogenously added acetone that is consumed to produce isobutene is roughly equal to the amount of acetone that is produced from glucose.

### Example 4: Production of Isobutene From Glucose

This example is meant to be illustrative but not inclusive of the various means to convert glucose to isobutene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass.

Cultures are started from frozen stock in shaker flasks and grown in medium until an OD 550 of approximately 1.0 A.U. is reached at which time the contents are transferred to the 1,000 liter seed fermenter. The microbes are further grown in the seed fermenter until they reach an OD 550 of approximately 10 A.U. at which time they are transferred into the 10,000 liter production fermenter

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C.

The pressure in the head space of the fermenter is maintained at 4 bar absolute throughout the course of the fermentation. The air flow rate is set in a range of 1,400 to 1,700 standard liters/minute of air over the course of the fermentation cycle.

Agitation intensity is maintained such as to ensure that the utilization of the oxygen fed to the fermenter is greater than 35%. Typically this means that the agitation intensity will be above 0.7 KW during the early stage of the fermentation cycle (ie microbe growth phase) and will rise to greater than 7 KW by the end of the fermentation cycle.

Due to isobutene's low solubility in water (about 260 ppm at 1 bar and 20°C), isobutene is continuously vaporized and continuously exits with the fermentation off-gases throughout the course of the fermentation. Isobutene does not accumulate to any appreciable extent in the fermentation liquid.

Due to acetone's higher boiling point, it will partly vent into the fermentation off-gases. Since the acetone conversion step is rather slow and a high rate depends on a high acetone concentration, acetone is recovered from the gaseous phase, i.e. the fermentation-off gas, and is continuously or semi-continuously reintroduced into the fermentation medium. The recovery of acetone from the fermentation-off gas can be achieved as shown in Figure 1. During the course of the initial microbe growth phase, acetone will accumulate to a 20 to 50 grams/liter concentration. An equilibrium between accumulation and consumption/venting will be reached and the acetone level will be maintained in this range during the rest of the fermentation cycle. The fermentation will continue until either contamination or the build-up of inhibitory compounds causes a notable decrease in overall isobutene production rate. This typically occurs in the 60th to 80^{th} hour of the cycle.

### Example 5: Fermentative production of isobutene from glucose (at 4 bar)

This example is meant to be illustrative but not inclusive of the various means to convert glucose to isobutene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass. Cultures are started from frozen stock in shaker flasks and grown in medium until an OD₅₅₀ of approximately 1.0 absorbance units (AU) is reached at which time the contents are transferred to a 1,000 liter seed fermenter. The microbes are further grown in the seed fermenter until they reach an OD₅₅₀ of approximately 10 AU at which time they are transferred into the 10,000 liter production fermenter.

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C. The pressure in the head space of the fermenter is maintained at 4 bar absolute throughout the course of the fermentation.

The air flow rate is set at such a rate that the exit oxygen concentration is always less than the about 13% level that is the flammable minimum oxygen concentration (MOC) value for isobutene in the presence of CO₂ and N₂ mixtures (Zabetakis MG, Flammability Characteristics of Combustible Gases and Vapors, U.S. Department of the Interior, Bureau of Mines, Bulletin 627, 1965, p. 52). Typically the exit concentration is maintained below 8%. Based on the chemistry of the reaction this corresponds to an air flow rate of less than 1,400 to 1,700 standard liters/minute of air at the end of the fermentation cycle. Agitation intensity is maintained such as to ensure that the utilization of the oxygen fed to the fermenter is greater than 35%. Typically this means that the agitation intensity will be about 0.7 KW during the early stage of the fermentation cycle (i.e., microbe growth phase) and will rise to 7 KW by the end of the fermentation cycle.

Due to isobutene's low solubility in water (about 260 ppm at 1 bar and 20°C), isobutene is continuously vaporized and continuously exits with the fermentation off-gases throughout the course of the fermentation. Isobutene does not accumulate to any appreciable extent in the fermentation liquid.

Due to acetone's higher boiling point, while it is also continuously venting into the fermentation off-gases, it also accumulates in the fermentation broth. Since the acetone conversion step is slow and a high rate depends on a high acetone concentration, this is advantageous to overall fermenter productivity. During the course of the initial microbe growth phase, acetone will accumulate to a 20 to 50 grams/liter concentration. An equilibrium between accumulation and consumption/venting will be reached and the acetone level will be maintained in this range during the rest of the fermentation cycle. The fermentation will continue until either contamination or the build-up of inhibitory compounds causes a notable decrease in overall isobutene production rate. This typically occurs in the 60th to 80th hour of the cycle.

The amount of acetone vented during the course of the fermentation cycle will depend on such factors as gas feed rate, fermenter temperature, fermenter absolute pressure and concentration of acetone in the broth. Typically 10 to 30% of the acetone produced will be vented or remain in the fermentation broth at the end of the fermentation cycle depending on the value of the aforementioned parameters. Clearly such a loss is uneconomical and so the vaporized acetone is recovered from the fermenter off-gases by well documented and understood technologies and continuously or semi-continuously returned to the fermenter during the course of the fermentation cycle to maintain a consistent acetone concentration in the broth. Any residual acetone in the broth at the end of fermentation cycle may be easily recovered by well documented and understood technologies for use in the next fermentation batch.

### Example 6: Fermentative production of 1,3-butadiene from glucose (at 3 bar)

This example is meant to be illustrative but not inclusive of the various means to convert glucose to 1,3-butadiene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass. Cultures are started from frozen stock in shaker flasks and grown in medium until an OD₅₅₀ of approximately 1.0 AU is reached at which time the contents are transferred to a 1,000 liter seed fermenter. The microbes are further grown in the seed fermenter until they reach an OD₅₅₀ of approximately 10 AU at which time they are transferred into the 10,000 liter production fermenter.

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C. The pressure in the head space of the fermenter is maintained at 3 bar absolute throughout the course of the fermentation.

The air flow rate is set at such a rate that the exit oxygen concentration is always less than the about 10.5% level that is the flammable minimum oxygen concentration (MOC) value for 1,3-butadiene in the presence of CO₂ and N₂ mixtures (Zabetakis MG, Flammability Characteristics of Combustible Gases and Vapors, U.S. Department of the Interior, Bureau of Mines, Bulletin 627, 1965, p. 55). Typically the exit concentration is maintained below 8%. Based on the chemistry of the reaction this corresponds to an air flow rate of less than 1,100 standard liters/minute of air at the end of the fermentation cycle. Agitation intensity is maintained such as to ensure that the utilization of the oxygen fed to the fermenter is greater than 45%. Typically this means that the agitation intensity will be about 0.4 KW during the early stage of the fermentation cycle (i.e., microbe growth phase) and will rise to 6 KW by the end of the fermentation cycle.

Due to 1,3-butadiene's low solubility in water (about 735 ppm at 1 bar and 20°C), 1,3-butadiene is continuously vaporized and continuously exits with the fermentation off-gases throughout the course of the fermentation. 1,3-butadiene does not accumulate to any appreciable extent in the fermentation liquid.

Due to crotyl alcohol's higher boiling point, while it is also continuously venting into the fermentation off-gases, it also accumulates in the fermentation broth. Since the crotyl alcohol conversion step is slow and a high rate depends on a high crotyl alcohol concentration, this is advantageous to overall fermenter productivity. During the course of the initial microbe growth phase, crotyl alcohol will accumulate to about 5 to 10 grams/liter concentration. An equilibrium between accumulation and consumption/venting will be reached and the crotyl alcohol level will be maintained in this range during the rest of the fermentation cycle. The fermentation will continue until either contamination or the build-up of inhibitory compounds causes a notable decrease in overall 1,3-butadiene production rate. This typically occurs in the 60th to 80th hour of the cycle.

The amount of crotyl alcohol vented during the course of the fermentation cycle will depend on such factors as gas feed rate, fermenter temperature, fermenter absolute pressure and concentration of crotyl alcohol in the broth. Typically 5 to 10% of the crotyl alcohol produced will be vented or remain in the fermentation broth at the end of the fermentation cycle depending on the value of the aforementioned parameters. Clearly such a loss is uneconomical and so the vaporized crotyl alcohol is recovered from the fermenter off-gases by well documented and understood technologies and continuously or semi-continuously returned to the fermenter during the course of the fermentation cycle to maintain a consistent crotyl alcohol concentration in the broth. Any residual crotyl alcohol in the broth at the end of fermentation cycle may be easily recovered by well documented and understood technologies for use in the next fermentation batch.

### Example 7: Fermentative production of 1,3-butadiene from glucose (at 2 bar)

This example is meant to be illustrative but not inclusive of the various means to convert glucose to 1,3-butadiene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass. Cultures are started from frozen stock in shaker flasks and grown in medium until an OD₅₅₀ of approximately 1.0 absorbance units (AU) is reached at which time the contents are transferred to a 1,000 liter seed fermenter. The microbes are further grown in the seed fermenter until they reach an OD₅₅₀ of approximately 10 AU at which time they are transferred into the 10,000 liter production fermenter.

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide and/or caustic addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C. The pressure in the head space of the fermenter is maintained at 2 bar absolute throughout the course of the fermentation.

The air flow rate is set at such a rate that the exit oxygen concentration is always less than the about 10.5% level that is the flammable minimum oxygen concentration (MOC) value for 1,3-butadiene in the presence of CO₂ and N₂ mixtures (Zabetakis MG, Flammability Characteristics of Combustible Gases and Vapors, U.S. Department of the Interior, Bureau of Mines, Bulletin 627, 1965, p. 55). Typically the exit concentration is maintained below 8%. Based on the chemistry of the reaction this corresponds to an air flow rate of less than 1,100 standard liters/minute of air at the end of the fermentation cycle. Agitation intensity is maintained such as to ensure that the utilization of the oxygen fed to the fermenter is greater than 45%. Typically this means that the agitation intensity will be about 0.4 KW during the early stage of the fermentation cycle (i.e., microbe growth phase) and will rise to 14 KW by the end of the fermentation cycle.

Due to 1,3-butadiene's low solubility in water (about 735 ppm at 1 bar and 20°C), 1,3-butadiene is continuously vaporized and continuously exits with the fermentation off-gases throughout the course of the fermentation. 1,3-butadiene does not accumulate to any appreciable extent in the fermentation liquid.

Due to crotyl alcohol's higher boiling point, while it is also continuously venting into the fermentation off-gases, it also accumulates in the fermentation broth. Since the crotyl alcohol conversion step is slow and a high rate depends on a high crotyl alcohol concentration, this is advantageous to overall fermenter productivity. During the course of the initial microbe growth phase, crotyl alcohol will accumulate to about 5 to 10 grams/liter concentration. An equilibrium between accumulation and consumption/venting will be reached and the crotyl alcohol level will be maintained in this range during the rest of the fermentation cycle. The fermentation will continue until either contamination or the build-up of inhibitory compounds causes a notable decrease in overall 1,3-butadiene production rate. This typically occurs in the 60th to 80th hour of the cycle.

The amount of crotyl alcohol vented during the course of the fermentation cycle will depend on such factors as gas feed rate, fermenter temperature, fermenter absolute pressure and concentration of crotyl alcohol in the broth. Typically 5 to 10% of the crotyl alcohol produced will be vented or remain in the fermentation broth at the end of the fermentation cycle depending on the value of the aforementioned parameters. Clearly such a loss is uneconomical and so the vaporized crotyl alcohol is recovered from the fermenter off-gases by well documented and understood technologies and continuously or semi-continuously returned to the fermenter during the course of the fermentation cycle to maintain a consistent crotyl alcohol concentration in the broth. Any residual crotyl alcohol in the broth at the end of fermentation cycle may be easily recovered by well documented and understood technologies for use in the next fermentation batch.

### Example 8: Fermentative production of propene from glucose (at 3 bar)

This example is meant to be illustrative but not inclusive of the various means to convert glucose to propene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass. Cultures are started from frozen stock in shaker flasks and grown in medium until an OD₅₅₀ of approximately 1.0 absorbance units (AU) is reached at which time the contents are transferred to a 1,000 liter seed fermenter. The microbes are further grown in the seed fermenter until they reach an OD₅₅₀ of approximately 10 AU at which time they are transferred into the 10,000 liter production fermenter.

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide and/or caustic addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C. The pressure in the head space of the fermenter is maintained at 3 bar absolute throughout the course of the fermentation.

The air flow rate is set at such a rate that the exit oxygen concentration is always less than the about 13% level that is the flammable minimum oxygen concentration (MOC) value for propylene in the presence of CO₂ and N₂ mixtures (Zabetakis MG, Flammability Characteristics of Combustible Gases and Vapors, U.S. Department of the Interior, Bureau of Mines, Bulletin 627, 1965, p. 51). Typically the exit concentration is maintained below 4%. Based on the chemistry of the reaction this corresponds to an air flow rate of less than 560 standard liters/minute of air at the end of the fermentation cycle. Agitation intensity is maintained such as to ensure that the utilization of the oxygen fed to the fermenter is greater than 55%. Typically this means that the agitation intensity will be about 0.4 KW during the early stage of the fermentation cycle (i.e., microbe growth phase) and will rise to 7.5 KW by the end of the fermentation cycle.

Due to propylene's low solubility in water (about 400 ppm at 1 bar and 20°C), propylene is continuously vaporized and continuously exits with the fermentation off-gases throughout the course of the fermentation. Propylene does not accumulate to any appreciable extent in the fermentation liquid.

Due to acetone's and isopropanol's higher boiling points, while they are also continuously venting into the fermentation off-gases, they also accumulate in the fermentation broth. Since the acetone and isopropanol conversion steps are not instantaneous, a high rate depends on high acetone and isopropanol concentrations; this is advantageous to overall fermenter productivity. During the course of the initial microbe growth and early production phases, acetone will accumulate to about 10-15 grams/liter concentration and isopropanol will accumulate to about 20-30 grams/liter concentration. An equilibrium between accumulation and consumption/venting will be reached and the acetone and isopropanol levels will be maintained in this range during the rest of the fermentation cycle.

The amount of acetone and isopropanol vented during the course of the fermentation cycle will depend on such factors as gas feed rate, fermenter temperature, fermenter absolute pressure and concentration of acetone and isopropanol in the broth. Typically 5 to 8% of the acetone and 8 to 12% of the isopropanol produced will be vented or remain in the fermentation broth at the end of the fermentation cycle depending on the value of the aforementioned parameters. Clearly such a loss is uneconomical and so the vaporized acetone and isopropanol are recovered from the fermenter off-gases by well documented and understood technologies and continuously or semi-continuously returned to the fermenter during the course of the fermentation cycle to maintain consistent acetone and isopropanol concentrations in the broth. Any residual acetone and isopropanol in the broth at the end of fermentation cycle may be easily recovered by well documented and understood technologies for use in the next fermentation batch.

### Example 9: Fermentative production of propene from glucose (at 10 bar)

This example is meant to be illustrative but not inclusive of the various means to convert glucose to propene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass. Cultures are started from frozen stock in shaker flasks and grown in medium until an OD₅₅₀ of approximately 1.0 absorbance units (AU) is reached at which time the contents are transferred to a 1,000 liter seed fermenter. The microbes are further grown in the seed fermenter until they reach an OD₅₅₀ of approximately 10 AU at which time they are transferred into the 10,000 liter production fermenter.

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide and/or caustic addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C. The pressure in the head space of the fermenter is maintained at 10 bar absolute throughout the course of the fermentation.

The air flow rate is set at such a rate that the exit oxygen concentration is always less than the about 13% level that is the flammable minimum oxygen concentration (MOC) value for propylene in the presence of CO₂ and N₂ mixtures (Zabetakis MG, Flammability Characteristics of Combustible Gases and Vapors, U.S. Department of the Interior, Bureau of Mines, Bulletin 627, 1965, p. 51). Typically the exit concentration is maintained below 1%. Based on the chemistry of the reaction this corresponds to an air flow rate of less than 380 standard liters/minute of air at the end of the fermentation cycle. Agitation intensity is maintained such as to ensure that the utilization of the oxygen fed to the fermenter is greater than 85%. Typically this means that the agitation intensity will be about 0.4 KW during the early stage of the fermentation cycle (i.e., microbe growth phase) and will rise to 6.6 KW by the end the end of the fermentation cycle.

Due to propylene's low solubility in water (about 400 ppm at 1 bar and 20°C), propylene is continuously vaporized and continuously exits with the fermentation off-gases throughout the course of the fermentation. Propylene does not accumulate to any appreciable extent in the fermentation liquid.

Due to acetone's and isopropanol's higher boiling points, while they are also continuously venting into the fermentation off-gases, they also accumulate in the fermentation broth. Since the acetone and isopropanol conversion steps are not instantaneous, a high rate depends on high acetone and isopropanol concentrations; this is advantageous to overall fermenter productivity. During the course of the initial microbe growth and early production phases, acetone will accumulate to about 10-15 grams/liter concentration and isopropanol will accumulate to about 20-30 grams/liter concentration. An equilibrium between accumulation and consumption/venting will be reached and the acetone and isopropanol levels will be maintained in this range during the rest of the fermentation cycle.

The amount of acetone and isopropanol vented during the course of the fermentation cycle will depend on such factors as gas feed rate, fermenter temperature, fermenter absolute pressure and concentration of acetone and isopropanol in the broth. Typically 4 to 6% of the acetone and 7 to 10% of the isopropanol produced will be vented or remain in the fermentation broth at the end of the fermentation cycle depending on the value of the aforementioned parameters. Clearly such a loss is uneconomical and so the vaporized acetone and isopropanol are recovered from the fermenter off-gases by well documented and understood technologies and continuously or semi-continuously returned to the fermenter during the course of the fermentation cycle to maintain consistent acetone and isopropanol concentrations in the broth. Any residual acetone and isopropanol in the broth at the end of fermentation cycle may be easily recovered by well documented and understood technologies for use in the next fermentation batch.

### Example 10: Fermentative production of propene from glucose (at 15 bar)

This example is meant to be illustrative but not inclusive of the various means to convert glucose to propene via fermentation. Materials and methods suitable for the maintenance and growth of bacterial cultures are well known in the art and will not be described in detail in this example. Techniques suitable for use can be found in Thomas D. Brock in Biotechnology: A Textbook of Industrial Microbiology, 2nd Edition (1989), Sinnauer Associates, Inc, Sunderland, Mass. Cultures are started from frozen stock in shaker flasks and grown in medium until an OD₅₅₀ of approximately 1.0 absorbance units (AU) is reached at which time the contents are transferred to a 1,000 liter seed fermenter. The microbes are further grown in the seed fermenter until they reach an OD₅₅₀ of approximately 10 AU at which time they are transferred into the 10,000 liter production fermenter.

Anti-foam, medium salts (containing phosphates, sulfate and citrates) and 6000 liters of water are sterilized in the production fermenter prior to the addition of the contents of the seed fermenter. pH is adjusted to 6.8 via ammonium hydroxide and/or caustic addition and enough concentrated glucose solution (60-70 wt%) is added to bring the glucose concentration to 10 grams/liter. Glucose concentration is maintained at 0 to 10 grams/liter throughout the fermentation. Temperature is controlled at 32°C. The pressure in the head space of the fermenter is maintained at 15 bar absolute throughout the course of the fermentation.

The air flow rate is set at such a rate that the exit oxygen concentration is always less than the about 13% level that is the flammable minimum oxygen concentration (MOC) value for propylene in the presence of CO₂ and N₂ mixtures (Zabetakis MG, Flammability Characteristics of Combustible Gases and Vapors, U.S. Department of the Interior, Bureau of Mines, Bulletin 627, 1965, p. 51). Typically the exit concentration is maintained below 1 %. Based on the chemistry of the reaction this corresponds to an air flow rate of less than 370 standard liters/minute of air at the end of the fermentation cycle. Agitation intensity is maintained such as to ensure that the utilization of the oxygen fed to the fermenter is greater than 85 %. Typically this means that the agitation intensity will be about 0.4 KW during the early stage of the fermentation cycle (i.e., microbe growth phase) and will rise to 4.2 KW by the end of the fermentation cycle.

Due to propylene's low solubility in water (about 400 ppm at 1 bar and 20°C), propylene is continuously vaporized and continuously exits with the fermentation off-gases throughout the course of the fermentation. Propylene does not accumulate to any appreciable extent in the fermentation liquid.

Due to acetone's and isopropanol's higher boiling points, while they are also continuously venting into the fermentation off-gases, they also accumulate in the fermentation broth. Since the acetone and isopropanol conversion steps are not instantaneous, a high rate depends on high acetone and isopropanol concentrations; this is advantageous to overall fermenter productivity. During the course of the initial microbe growth and early production phases, acetone will accumulate to about 10-15 grams/liter concentration and isopropanol will accumulate to about 20-30 grams/liter concentration. An equilibrium between accumulation and consumption/venting will be reached and the acetone and isopropanol levels will be maintained in this range during the rest of the fermentation cycle.

The amount of acetone and isopropanol vented during the course of the fermentation cycle will depend on such factors as gas feed rate, fermenter temperature, fermenter absolute pressure and concentration of acetone and isopropanol in the broth. Typically 4 to 6% of the acetone and 7 to 10% of the isopropanol produced will be vented or remain in the fermentation broth at the end of the fermentation cycle depending on the value of the aforementioned parameters. Clearly such a loss is uneconomical and so the vaporized acetone and isopropanol are recovered from the fermenter off-gases by well documented and understood technologies and continuously or semi-continuously returned to the fermenter during the course of the fermentation cycle to maintain consistent acetone and isopropanol concentrations in the broth. Any residual acetone and isopropanol in the broth at the end of fermentation cycle may be easily recovered by well documented and understood technologies for use in the next fermentation batch.

### Example 11: Assessment of the optimal concentration of acetone for Isobutene (IBN) production

### Media

LB is Luria Bertani medium made of tryptone 10 g/l, yeast extract 5 g/l and NaCl 10 g/l ZYM-5052 medium is described in FW Studier (2005) Prot. Exp. Pur. 41, 207-234.

MS medium is described in C. Richaud et al. (1993) J. Biol. Chem. Vol. 268, N° 36, pp 26827-26835.

MSP medium is a MS medium with 200 mM di-potassium phosphate instead of 50 mM. MSP 45 medium is an MSP medium which has been adjusted to pH 8.5 and contains 45 g/l glucose instead of 2.

Recombinant E. coli strains expressing genes for isobutene production (WO 2010/001078 A2, WO 2011/032934 A1) are isolated on LB plate + 100 mg/l ampicillin. One colony is grown in 10 ml LB medium + 100 mg/l ampicillin for one night at 30°C. Then 300 ml of ZYM-5052 medium + 100 mg/l ampicillin are inoculated with 1 ml of the previous culture and grown at 30°C for 24h. Such cells are collected by centrifugation at 4000 g, then suspended in MSP45 medium + 100 mg/l ampicillin to reach an optical density at 600nm around 25. Finally 30 ml of the suspension are introduced in a 160 ml bottle and just after acetone (0.1 to 1 M final concentration) is added. The bottle is then sealed and incubated on a shaker at 30°C. After 6h incubation period, a sample is taken to measure 3-hydroxy-isovaleric acid (HIV) content by HPLC and pH is adjusted back to 8.5 with ammonia. Samples of gas are taken after 2, 4, 6 or 24 hours of incubation to measure the IBN production by Gas Chromatography.

The following tables gather the results of the production kinetics of HIV and isobutene at various acetone concentrations in sealed bottles.

**Table 1**

| **[acetone] mM** | **0** | **100** | **200** | **400** | **600** | **800** | **1000** |
|---|---|---|---|---|---|---|---|
| **HIV at 6h** | 0 | + | ++ | +++ | +++/++++ | ++++ | ++++ |
| **IBN at 6h** | 0 | ++ | +++ | +++/++++ | +++++ | +++++++ | +++ |

| | | | | | | | |
|---|---|---|---|---|---|---|---|
| HIV: 3-hydroxy-isovaleric acid IBN: Isobutene | | | | | | | |

**Table 2**

| **[acetone] mM** | **0** | **100** | **200** | **400** | **600** | **800** | **1000** |
|---|---|---|---|---|---|---|---|
| **IBN at 6h** | 0 | +/- | + | + | +/++ | ++ | + |
| **IBN at 24h** | 0 | +/++ | +++ | ++++ | ++++++ | +++++ | ++++++ |

| | | | | | | | |
|---|---|---|---|---|---|---|---|
| HIV: 3-hydroxy-isovaleric acid IBN: Isobutene | | | | | | | |

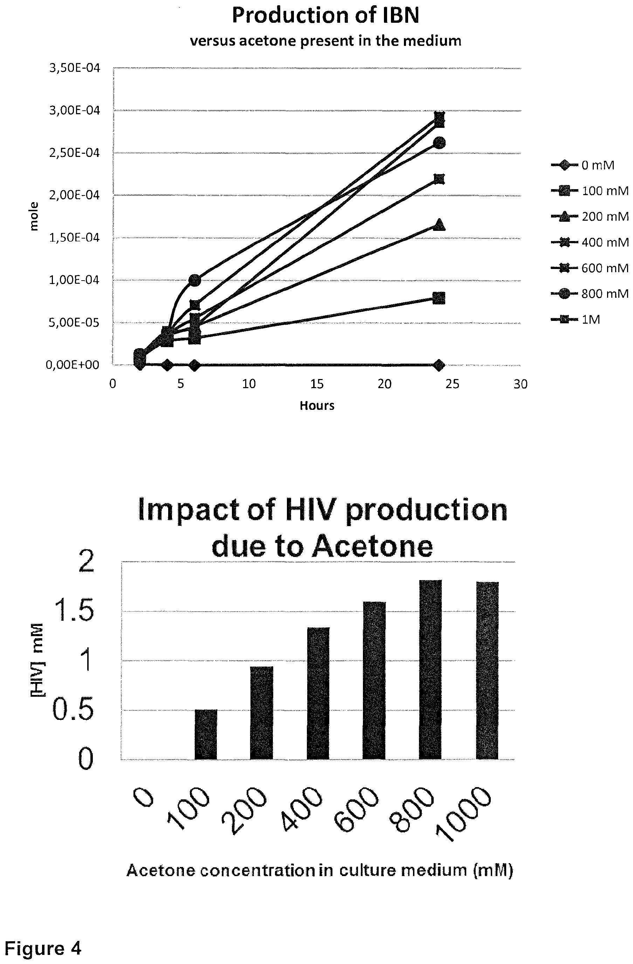

The results are also illustrated in Figure 4. First, it is noticeable that there is no or only traces of production of either HIV or IBN in the absence of acetone. Second, both HIV and IBN productions increase with the acetone concentration up to an optimal level around 600 mM. The advantage of the sealed bottle is that the acetone concentration decrease is only due to the consumption rate for HIV and IBN production and it is rather negligible in the experiment described.

The situation is quite different in an aerated bioreactor since a more significant portion of acetone will be removed by stripping, depending of the temperature, the pressure and the aeration rate.

For instance, in a 900 ml culture run at 30°C and with an aeration rate of 0.3 L/min under standard pressure, the 600 mM initial acetone concentration decreases steadily and there is almost no acetone remaining in the bioreactor after 2 days.

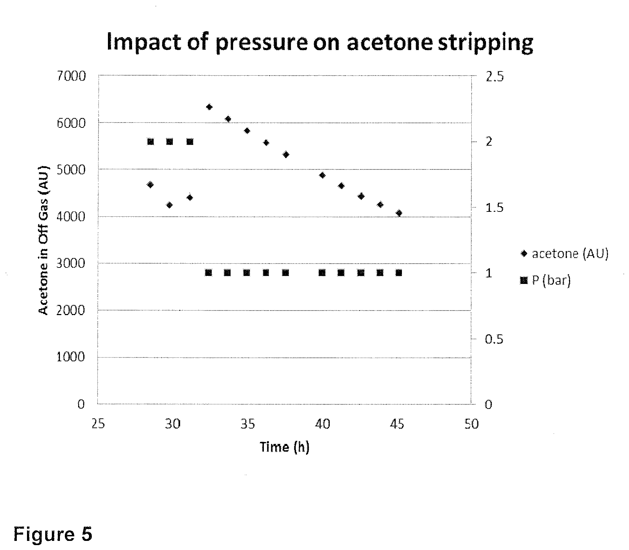

An E. coli culture has been run in the presence of 100 mM acetone at 30°C with an aeration rate of 7L/min either at standard pressure or at 2 bars absolute pressure. The acetone content has been measured either in the culture by HPLC or in the off gas stream with on line gas chromatography. The results are shown in Figure 5. There is a very significant increase in the amount of acetone stripped in the off gas when the pressure is decreased from 2 bars to atmospheric pressure at 32 hours.

## Claims

1. A fermentation method for producing a hydrocarbon compound comprising the culturing of an organism in a liquid fermentation medium, wherein said organism produces a desired hydrocarbon compound by an enzymatic pathway, said enzymatic pathway comprising an intermediate which evaporates into the gaseous phase and wherein said intermediate is recovered from the gaseous phase and is reintroduced into the liquid fermentation medium, and wherein said intermediate is selected from an aldehyde, a ketone and an alcohol.

2. The fermentation method of claim 1, wherein said intermediate is **characterized in that** when present as a pure compound and/or as a potential compound-water azeotrope of the intermediate it has an apparent boiling point of less than 120 °C at 1.013 bar or **in that** the apparent vapour pressure of the intermediate at infinite dilution in water at 25°C, which is defined as the pure component vapour pressure multiplied by the water-intermediate activity coefficient at infinite water dilution, is greater than 10 millibar.

3. The fermentation method of claim 1 or 2, wherein said intermediate is selected from the group consisting of acetaldehyde, ethanol, acetone, propanal, propan-1-ol, propan-2-ol, butanal, butan-1-ol, butanone, butan-2-ol, 2-methyl-propanal, 2-methyl-propan-1-ol, 2-buten-1-ol (crotyl alcohol), 3-buten-2-ol, 3-methyl-2-buten-1-ol (prenol), 2-methyl-3-buten-2-ol, 3-methyl-3-buten-1-ol (isoprenol) and 3-methyl-3-buten-2-ol.

4. The fermentation method of claim 3, wherein the hydrocarbon is a C₂₋₈ alkene of the following formula (I): 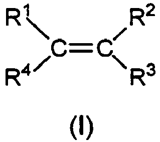 wherein R¹, R², R³ and R⁴ are each independently selected from hydrogen, C₁₋₆ alkyl or C₂₋₆ alkenyl, provided that the total number of carbon atoms in the compound of formula (I) is an integer of 2 to 8.

5. The fermentation method of claim 3 or 4, wherein the hydrocarbon is selected from the group consisting of isobutene, 1,3-butadiene, isoprene (2-methyl-1,3-butadiene) or propene.

6. The fermentation method of any one of claims 1 to 5, wherein said intermediate is acetone.

7. The fermentation method of claim 6, wherein the desired hydrocarbon compound to be produced is isobutene and the enzymatic pathway leading to the production of isobutene comprises acetone as an intermediate.

8. The fermentation method of claim 7, wherein the enzymatic pathway for the production of isobutene comprises the conversion of acetone and acetyl-CoA into 3-hydroxy-3-methylbutyric acid.

9. The fermentation method of claim 8, wherein the enzymatic conversion of acetone into 3-hydroxy-3-methylbutyric acid is achieved by the use of an enzyme having the activity of an HMG CoA synthase (EC 2.3.3.10) or an enzyme having the activity of a C-C bond cleavage/condensation lyase, such as a HMG CoA lyase (EC 4.1.3.4), or a PksG protein.

10. The fermentation method of claim 6, wherein the desired hydrocarbon compound to be produced is propylene and the enzymatic pathway leading to the production of propylene comprises acetone as an intermediate.

11. The fermentation method of claim 10, wherein the enzymatic pathway for the production of propylene comprises the conversion of acetone into propane-2-ol.

12. The fermentation method of any one of claims 1 to 11, wherein the organism is a microorganism.

13. The fermentation method of claim 12, wherein the microorganism is a bacterium.

14. The fermentation method of claim 13, wherein the bacterium is E. coli.

15. The fermentation method of any one of claims 1 to 14, wherein the intermediate is recovered from the gaseous phase by
a. Physical absorption with or without distillation;
b. Reactive absorption with or without distillation;
c. Adsorption;
d. Condensation;
e. Cryogenic technologies; and
f. Membrane based separations.

16. The fermentation method of any one of claims 1 to 15, wherein said fermentation method is carried out as a batch method.

17. The fermentation method of any one of claims 1 to 16, wherein said fermentation method is carried out under increased pressure.

## Patentansprüche

1. Fermentationsverfahren zur Herstellung einer Kohlenwasserstoffverbindung, umfassend das Züchten eines Organismus in einem flüssigen Fermentationsmedium, wobei der Organismus die gewünschte Kohlenwasserstoffverbindung mittels eines enzymatischen Wegs herstellt, wobei der enzymatische Weg ein Zwischenprodukt umfasst, das in die Gasphase evaporiert und wobei das Zwischenprodukt aus der Gasphase zurückgewonnen und wieder in das flüssige Fermentationsmedium eingeführt wird, und wobei das Zwischenprodukt ausgewählt ist aus einem Aldehyd, einem Keton und einem Alkohol.

2. Fermentationsverfahren nach Anspruch 1, wobei das Zwischenprodukt **dadurch gekennzeichnet ist, dass**, wenn als reine Verbindung und/oder als ein potenzielles Verbindung-Wasser-Azeotrop des Zwischenprodukts vorliegt, es einen scheinbaren Siedepunkt von weniger als 120°C bei 1,013 bar hat, oder, dass der scheinbare Dampfdruck des Zwischenprodukts bei unendlicher Verdünnung im Wasser bei 25°C, der definiert ist als Reinstoff-Dampfdruck multipliziert durch den Wasser-Zwischenprodukt-Aktivitäts-Koeffizienten bei unendlicher Wasserverdünnung, größer als 10 Millibar ist.

3. Fermentationsverfahren nach Anspruch 1 oder 2, wobei das Zwischenprodukt ausgewählt ist aus der Gruppe bestehend aus Acetaldehyd, Ethanol, Aceton, Propanal, Propan-1-ol, Propan-2-ol, Butanal, Butan-1-ol, Butanon, Butan-2-ol, 2-Methylpropanal, 2-Methylpropan-1-ol, 2-Buten-1-ol (Crotylalkohol), 3-Buten-2-ol, 3-Methyl-2-buten-1-ol (Prenol), 2-Methyl-3-buten-2-ol, 3-Methyl-3-buten-1-ol (Isoprenol) und 3-Methyl-3-buten-2-ol.

4. Fermentationsverfahren nach Anspruch 3, wobei der Kohlenwasserstoff ein C₂₋₈-Alken der folgenden Formel (I) ist: 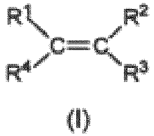 wobei R¹, R², R³ und R⁴ jedes unabhängig ausgewählt ist aus Wasserstoff, C₁₋₆-Alkyl oder C₂₋₆-Alkenyl, mit der Maßgabe, dass die Gesamtanzahl der Kohlenstoffatome in der Verbindung der Formel (I) eine ganze Zahl von 2 bis 8 ist.

5. Fermentationsverfahren nach Anspruch 3 oder 4, wobei der Kohlenstoff ausgewählt ist aus der Gruppe bestehend aus Isobuten, 1,3-Butadien, Isopren (2-Methyl-1,3-butadien) oder Propen.

6. Fermentationsverfahren nach einem der Ansprüche 1 bis 5, wobei das Zwischenprodukt Aceton ist.

7. Fermentationsverfahren nach Anspruch 6, wobei die gewünschte herzustellende Kohlenstoffverbindung Isobuten ist und der enzymatische Weg, der zur Herstellung von Isobuten führt, Aceton als Zwischenprodukt umfasst.

8. Fermentationsverfahren nach Anspruch 7, wobei der enzymatische Weg zur Herstellung von Isobuten, die Umsetzung von Aceton und Acetyl-CoA in 3-Hydroxy-3-methylbuttersäure umfasst.

9. Fermentationsverfahren nach Anspruch 8, wobei die enzymatische Umsetzung von Aceton in 3-Hydroxy-3-methylbuttersäure erreicht wird durch die Verwendung eines Enzyms, das die Aktivität einer HMG-CoA-Synthase (EC 2.3.3.10) aufweist, oder eines Enzyms, das die Aktivität einer C-C-Bindungsspaltung/Kondensationslyase, wie eine HMG-CoA-Lyase (EC 4.1.3.4), aufweist, oder eines PksG-Proteins.

10. Fermentationsverfahren nach Anspruch 6, wobei die gewünschte herzustellende Kohlenstoffverbindung Propylen ist und der enzymatische Weg, der zur Herstellung von Propylen führt, Aceton als Zwischenprodukt umfasst.

11. Fermentationsverfahren nach Anspruch 10, wobei der enzymatische Weg zur Herstellung von Propylen die Umsetzung von Aceton in Propan-2-ol umfasst.

12. Fermentationsverfahren nach einem der Ansprüche 1 bis 11, wobei der Organismus ein Mikroorganismus ist.

13. Fermentationsverfahren nach Anspruch 12, wobei der Mikroorganismus ein Bakterium ist.

14. Fermentationsverfahren nach Anspruch 13, wobei das Bakterium *E*. *coli* ist.

15. Fermentationsverfahren nach einem der Ansprüche 1 bis 14, wobei das Zwischenprodukt aus der Gasphase gewonnen wird durch
a. physische Absorption mit oder ohne Destillation;
b. reaktive Absorption mit oder ohne Destillation;
c. Adsorption;
d. Kondensation;
e. kryogene Techniken; und
f. Membran-basierte Trennungen.

16. Fermentationsverfahren nach einem der Ansprüche 1 bis 15, wobei das Fermentationsverfahren als Batch-Verfahren durchgeführt wird.

17. Fermentationsverfahren nach einem der Ansprüche 1 bis 16, wobei das Fermentationsverfahren unter erhöhtem Druck durchgeführt wird.

## Revendications

1. Méthode de fermentation pour produire un composé hydrocarboné, comprenant la culture d'un organisme dans un milieu de fermentation liquide, dans laquelle ledit organisme produit un composé hydrocarboné souhaité par une voie enzymatique, ladite voie enzymatique comprenant un intermédiaire qui s'évapore dans la phase gazeuse et dans laquelle ledit intermédiaire est récupéré de la phase gazeuse et est réintroduit dans le milieu de fermentation liquide, et dans laquelle ledit intermédiaire est choisi parmi un aldéhyde, une cétone et un alcool.

2. Méthode de fermentation selon la revendication 1, dans laquelle ledit intermédiaire est **caractérisé en ce que**, lorsqu'il est présent sous la forme d'un composé pur et/ou sous la forme d'un azéotrope composé-eau potentiel de l'intermédiaire, il a un point d'ébullition apparent inférieur à 120 °C sous 1,013 bar, ou **en ce que** la pression de vapeur apparente de l'intermédiaire à une dilution infinie dans de l'eau à 25 °C, qui est définie comme la pression de vapeur du composant pur multipliée par le coefficient d'activité de l'eau-intermédiaire à une dilution dans l'eau infinie, est supérieure à 10 millibars.

3. Méthode de fermentation selon la revendication 1 ou 2, dans laquelle ledit intermédiaire est choisi dans le groupe constitué par l'acétaldéhyde, l'éthanol, l'acétone, le propanal, le propan-1-ol, le propan-2-ol, le butanal, le butan-1-ol, la butanone, le butan-2-ol, le 2-méthylpropanal, le 2-méthylpropan-1-ol, le 2-butén-1-ol (alcool crotylique), le 3-butén-2-ol, le 3-méthyl-2-butén-1-ol (prénol), le 2-méthyl-3-butén-2-ol, le 3-méthyl-3-butén-1-ol (isoprénol) et le 3-méthyl-3-butén-2-ol.

4. Méthode de fermentation selon la revendication 3, dans laquelle l'hydrocarbone est un alcène en C₂ à C₈ de formule (I) suivante : 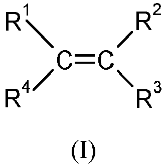 dans laquelle chacun de R¹, R², R³ et R⁴ est indépendamment choisi parmi un hydrogène, alkyle en C₁ à C₆ ou alcényle en C₂ à C₆, sous réserve que le nombre total d'atomes de carbone dans le composé de formule (I) soit un entier de 2 à 8.

5. Méthode de fermentation selon la revendication 3 ou 4, dans laquelle l'hydrocarbone est choisi dans le groupe constitué par l'isobutène, le 1,3-butadiène, l'isoprène (2-méthyl-1,3-butadiène) ou le propène.

6. Méthode de fermentation selon l'une quelconque des revendications 1 à 5, dans laquelle ledit intermédiaire est l'acétone.

7. Méthode de fermentation selon la revendication 6, dans laquelle le composé hydrocarboné souhaité devant être produit est l'isobutène et la voie enzymatique conduisant à la production d'isobutène comprend de l'acétone en tant qu'intermédiaire.

8. Méthode de fermentation selon la revendication 7, dans laquelle la voie enzymatique pour la production d'isobutène comprend la conversion d'acétone et d'acétyl-CoA en acide 3-hydroxy-3-méthylbutyrique.

9. Méthode de fermentation selon la revendication 8, dans laquelle la conversion enzymatique d'acétone en acide 3-hydroxy-3-méthylbutyrique est réalisée par l'utilisation d'une enzyme ayant l'activité d'une HMG CoA synthase (EC 2.3.3.10) ou d'une enzyme ayant l'activité d'une lyase de condensation/coupure de liaison C-C telle qu'une HMG CoA lyase (EC 4.1.3.4), ou d'une protéine PksG.

10. Méthode de fermentation selon la revendication 6, dans laquelle le composé hydrocarboné souhaité devant être produit est le propylène et la voie enzymatique conduisant à la production de propylène comprend de l'acétone en tant qu'intermédiaire.

11. Méthode de fermentation selon la revendication 10, dans laquelle la voie enzymatique pour la production de propylène comprend la conversion d'acétone en propan-2-ol.

12. Méthode de fermentation selon l'une quelconque des revendications 1 à 11, dans laquelle l'organisme est un microorganisme.

13. Méthode de fermentation selon la revendication 12, dans laquelle le microorganisme est une bactérie.

14. Méthode de fermentation selon la revendication 13, dans laquelle la bactérie est E. coli.

15. Méthode de fermentation selon l'une quelconque des revendications 1 à 14, dans laquelle l'intermédiaire est récupéré de la phase gazeuse par
a. absorption physique avec ou sans distillation ;
b. absorption réactive avec ou sans distillation ;
c. adsorption ;
d. condensation ;
e. des technologies cryogéniques ; et
f. des séparations basées sur une membrane.

16. Méthode de fermentation selon l'une quelconque des revendications 1 à 15, dans laquelle ladite méthode de fermentation est mise en œuvre comme une méthode en discontinu.

17. Méthode de fermentation selon l'une quelconque des revendications 1 à 16, dans laquelle ladite méthode de fermentation est mise en œuvre sous une pression augmentée.
